(19) Europäisches Patentamt
European Patent Office
Office européen des brevets

(11) **EP 4 265 595 A1**

(12) **EUROPEAN PATENT APPLICATION**
published in accordance with Art. 153(4) EPC

(43) Date of publication:
**25.10.2023 Bulletin 2023/43**

(21) Application number: **21910746.3**

(22) Date of filing: **21.12.2021**

(51) International Patent Classification (IPC):
**C07C 303/44** (2006.01)   **C07C 303/20** (2006.01)
**C07C 303/32** (2006.01)   **C07C 309/17** (2006.01)
**C09K 23/10** (2022.01)   **C11D 1/28** (2006.01)
**C11D 3/20** (2006.01)   **C11D 3/43** (2006.01)

(52) Cooperative Patent Classification (CPC):
**C07C 303/20; C07C 303/32; C07C 303/44;
C07C 309/17; C09K 23/10; C11D 1/28; C11D 3/20;
C11D 3/43**

(86) International application number:
**PCT/JP2021/047178**

(87) International publication number:
**WO 2022/138603 (30.06.2022 Gazette 2022/26)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **21.12.2020 JP 2020211482**

(71) Applicant: **Kao Corporation
Chuo-ku
Tokyo 103-8210 (JP)**

(72) Inventors:
• **MITSUDA Yoshinori
  Wakayama-shi, Wakayama 640-8580 (JP)**
• **CHIBA Keisuke
  Wakayama-shi, Wakayama 640-8580 (JP)**
• **NOMURA Masato
  Wakayama-shi, Wakayama 640-8580 (JP)**

(74) Representative: **Hoffmann Eitle
Patent- und Rechtsanwälte PartmbB
Arabellastraße 30
81925 München (DE)**

(54) **SULFONATE COMPOSITION PRODUCTION METHOD**

(57) A method for producing a sulfonate composition including, reacting a diester of a predetermined unsaturated carboxylic acid [hereinafter referred to as component (A)] in the presence of one or more solvents selected from (B) (B1) an organic solvent [hereinafter referred to as component (B1)] and (B2) water [hereinafter referred to as component (B2)] with (C) a sulfonating agent to obtain a mixture containing (S) a predetermined sulfonate having a diester structure [hereinafter referred to as component (S)], and removing an insoluble component from the mixture, wherein in the mixture from which the removal of the insoluble component is made, the content of component (B1) to a total content of components (S), (B1) and (B2) is 0.05 or more and 0.65 or less, and the content of component (B2) to the total content of components (S), (B1) and (B2) is 0.03 or more and 0.26 or less.

EP 4 265 595 A1

**Description**

Field of the Invention

The present invention relates to a method for producing a sulfonate composition.

Background of the Invention

Some sulfonates such as sulfosuccinate esters or the like are used as anionic surfactants, and utilized alone or in combination with other components as detergent bases, emulsifiers, wetting agents, dispersants or the like in various fields.

US-A 2007/0214999 discloses a compound comprising a salt of a mono- and/or dialkyl ester of a sulfonated dicarboxylic acid, wherein the dicarboxylic acid contains 4 to 8 carbon atoms and the alkyl groups are derived from 2-propylheptanol, and a composition comprising this compound and a predetermined organic solvent.

JP-A 2002-224552 discloses an emulsifying and dispersing agent obtained by using a combination of two or more types of anionic surfactants (A) selected from the group consisting of the following: (A1) sulfate esters of a predetermined adduct of an aliphatic alcohol and an alkylene oxide, (A2) sulfosuccinate esters of the adduct, (A3) ethers of a carboxylate and the adduct and (A4) phosphate esters of the adduct.

JP-A H9-500884 discloses a liquid composition comprising, in water as a solvent or a cosolvent, a sulfosuccinic acid diester represented by a specific formula, wherein the cosolvent is a polymer which contains alkylene oxide units and is a liquid at 10°C.

JP-A H8-337567 discloses a method for producing a sulfosuccinate ester comprising, adding an inorganic acid salt or an organic acid salt to an aqueous solution of the sulfosuccinate ester, and cooling after heating this aqueous solution to remove a deposit.

JP-A 2018-162446 discloses a method for producing a surfactant composition comprising, a step of converting an esterified product (C) of an unsaturated dicarboxylic acid having 4 to 22 carbons (c1) and a fatty acid monoalcohol having 1 to 22 carbons (c2) with a sulfonating agent (D) to a sulfonated product (E).

Summary of the Invention

It is considered that surfactants worth utilizing among sulfonates are diester-type sulfonates having an alkyl group and/or alkenyl group with a predetermined number of carbons because they have desirable foam behavior such as formation of fine foam when they are used in aqueous solutions and rapid foam disappearance, high cleaning power against solid fat stains, and others.

Sulfonates are generally produced using aqueous reaction media, and obtained as reaction products including water. Considering distribution costs or the like, it is desirable that the reaction products from which water and other components are reduced to increase the concentration of a sulfonate composition of the main component to a certain degree be used for the preparation of secondary products. For these reasons, a sulfonate composition containing a sulfonate at a higher degree of purity is preferred, and there is a need for a technology that provides a sulfonate composition having a low content of components other than a sulfonate (for example, inorganic salts or the like).

The present invention provides a sulfonate composition containing a diester-type sulfonate having an alkyl group and/or alkenyl group with a predetermined number of carbons and having a low content of inorganic metal compounds such as inorganic salts or the like.

The present invention relates to a method for producing a sulfonate composition including, reacting (A) a compound represented by the following general formula (1) [hereinafter referred to as component (A)] in the presence of one or more solvents [hereinafter referred to as component (B)] selected from (B) (B1) an organic solvent [hereinafter referred to as component (B1)] and (B2) water [hereinafter referred to as component (B2)] with (C) a sulfonating agent [hereinafter referred to as component (C)] to obtain a mixture containing (S) a sulfonate represented by the following general formula (2) [hereinafter referred to as component (S)], and removing an insoluble component from the mixture,

$$ROOC\text{-}(CH_2)_n\text{-}CH=CH\text{-}(CH_2)_m\text{-}COOR' \qquad (1)$$

wherein R and R' independently represent a group selected from alkyl groups with 10 carbons and alkenyl groups with 10 carbons; and n and m each represent a number of 0 or more and 18 or less, and a total of n and m is 0 or more and 18 or less,

$$ROOC\text{-}(CH_2)_p\text{-}CH(SO_3M)\text{-}CH_2\text{-}(CH_2)_q\text{-}COOR' \qquad (2)$$

wherein M represents a cation, and R and R' independently represent a group selected from alkyl groups with 10 carbons and alkenyl groups with 10 carbons; and p and q each represent a number of 0 or more and 18 or less, and a total of p and q is 0 or more and 18 or less,

wherein the mixture from which the removal of the insoluble component is made contains components (S), (B1) and (B2), wherein a mass ratio of the content of component (B1) to a total content of components (S), (B1) and (B2), (B1)/(S + B1 + B2), is 0.05 or more and 0.65 or less, and a mass ratio of the content of component (B2) to the total content of components (S), (B1) and (B2), (B2)/(S + B1 + B2), is 0.03 or more and 0.26 or less.

The method for producing a sulfonate composition of the present invention can produce a sulfonate composition containing a diester-type sulfonate having an alkyl group and/or alkenyl group with a predetermined number of carbons and having a low content of inorganic metal compounds such as inorganic salts or the like.

The method for producing a sulfonate composition of the present invention is suitable for the production of a sulfonate composition including a high concentration of a diester-type sulfonate having an alkyl group and/or alkenyl group with a predetermined number of carbons and excellent in storage stability.

Embodiments of the Invention

Diligent study to solve the aforementioned problem has led the present inventors to reach an invention of a method for producing a sulfonate composition having a low content of inorganic metal compounds in the sulfonate. A sulfonate composition having a low content of inorganic metal compounds in the sulfonate can be obtained by the producing method of the present invention. Further, as a result of the diligent study, the present inventors have found that the storage stability of the sulfonate composition is affected by an increase in the content of inorganic metal compounds such as inorganic salts or the like in the sulfonate composition. Note that a composition including a high concentration of a sulfonate and having a low content of inorganic metal compounds such as inorganic salts or the like also has secondary effects that increase the degree of freedom in formulation, for example, excellent low-temperature stability when it is used to formulate detergent compositions, or the like.

In general, sulfonates are produced through a reaction of sulfonating a compound represented by the general formula (1). This reaction produces a mixture including a sulfonate, and insoluble components such as inorganic salts or the like are removed from this mixture.

It is inferred that, in the present invention, when the insoluble component is removed from the mixture including the sulfonate, the deposition of inorganic metal compounds such as inorganic salts or the like from the mixture is promoted if the contents of organic solvent (B1) and water (B2) in the mixture from which the removal of the insoluble component is made fall within the predetermined ranges. Further, it is inferred that removing the insoluble component from the mixture in which a large amount of inorganic metal compounds such as inorganic salts or the like is deposited leads to a reduced content of inorganic metal compounds such as inorganic salts or the like in the mixture after the removal of the insoluble component.

The present invention relates to a method for producing a sulfonate composition including, reacting (A) a compound represented by the general formula (1) [hereinafter referred to as component (A)] in the presence of one or more solvents [hereinafter referred to as component (B)] selected from (B) (B1) an organic solvent [hereinafter referred to as component (B1)] and (B2) water [hereinafter referred to as component (B2)] with (C) a sulfonating agent [hereinafter referred to as component (C)] under predetermined conditions to obtain a mixture containing (S) a sulfonate represented by the general formula (2) [hereinafter referred to as component (S)], and removing an insoluble component from the mixture. The insoluble component is, for example, a solid in the mixture. This solid may be a solid existing while not dissolved in the mixture or a solid deposited from the mixture by an operation carried out after the step of obtaining the mixture.

First, component (A), a raw material used for the sulfonation reaction of component (A), and components (B) and (C) used for the sulfonation reaction of component (A) are explained.

Component (A) is a compound represented by the following general formula (1):

$$\text{ROOC-(CH}_2\text{)}_n\text{-CH=CH-(CH}_2\text{)}_m\text{-COOR'} \qquad (1)$$

wherein R and R' independently represent a group selected from alkyl groups with 10 carbons and alkenyl groups with 10 carbons; and n and m each represent a number of 0 or more and 18 or less, and a total of n and m is 0 or more and 18 or less.

R and R' independently represent a group selected from alkyl groups with 10 carbons and alkenyl groups with 10 carbons. R and R' independently represent preferably a group selected from branched-chain alkyl groups with 10 carbons and branched-chain alkenyl groups with 10 carbons, and more preferably a branched-chain alkyl group with 10 carbons from the viewpoint of washing performance. Examples of the alkyl groups with 10 carbons include n-decyl group, iso-decyl group, 2-propylheptyl group, 4-methyl 2-propylhexyl group and 5-methyl 2-propylhexyl group. Examples of the alkenyl groups with 10 carbons include n-decenyl group and iso-decenyl group. R and R' independently represent further preferably a group selected from 2-propylheptyl group, 4-methyl 2-propylhexyl group and 5-methyl 2-propylhexyl group from the viewpoint of washing performance.

n and m each represent 0 or more, and may each represent 18 or less, 16 or less, 12 or less, 8 or less, 4 or less, 2 or less, 1 or less or 0. Further, the total of n and m is 0 or more, and may be 18 or less, 16 or less, 12 or less, 8 or less, 4 or less, 2 or less, 1 or less or 0.

In the present invention, one or two or more types of components (A) can be used. In the method for producing a sulfonate composition of the present invention, a compound for which one of or both R and R' represent 2-propylheptyl group is preferably used as component (A) from the viewpoint of the washing performance of a sulfonate composition obtained by the method for producing a sulfonate composition of the present invention [hereinafter referred to as the sulfonate composition of the present invention].

Component (A) is preferably an ester of an unsaturated dicarboxylic acid with 4 or more and 22 or less carbons, for example, preferably a maleic acid diester, a fumaric acid diester, a citraconic acid diester, a mesaconic acid diester, a 2-pentenedioic acid diester, a methylene succinic acid diester, an allylmalonic acid diester, an isopropylidene succinic acid diester, a 2,4-hexadienedioic acid diester and an acetylenedicarboxylic acid diester, and more preferably maleic acid and fumaric acid diesters from the viewpoints of the permeability, washability and formulation stability of the sulfonate composition of the present invention.

Component (B) is a solvent. Component (B) is a solvent including one or more selected from components (B1) and (B2).

Component (B1) is an organic solvent. Examples of component (B1) include at least one selected from a monohydric alcohol and a polyhydric alcohol. Considering uses of the sulfonate composition of the present invention, for example, a use as a raw material for producing household products, component (B1) is preferably one with less odor, and preferably a compound approved as a food additive, a cosmetic raw material or the like.

The monohydric alcohol may have 1 or more and 12 or less carbons. Examples of the monohydric alcohol include methanol, ethanol, 1-propanol, 1-butanol, 1-pentanol, 1-hexanol, 1-octanol, 1-nonanol, 2-nonanol, 5-nonanol, 1-decanol, 1-dodecanol, 3,5,5-trimethylhexan-1-ol, 2,6-dimethyl-4-heptanol, 2-propylheptanol, 4-methyl 2-propylhexanol, 5-methyl 2-propylhexanol or the like. The monohydric alcohol is preferably a compound selected from ethanol, 1-butanol and 1-propanol, and more preferably ethanol from the viewpoints of uniformity in appearance and use as a versatile raw material.

The polyhydric alcohol may have 2 or more and 12 or less carbons. The polyhydric alcohol may be dihydric or more and hexahydric or less. Examples of the polyhydric alcohol include ethylene glycol, propylene glycol, 1,3-butanediol, 1,4-butanediol, 1,5-pentanediol, 1,6-hexanediol, 1,10-decanediol, diethylene glycol, triethylene glycol, tetraethylene glycol, dipropylene glycol, tripropylene glycol, glycerin, xylitol, sorbitol or the like. The polyhydric alcohol is preferably a compound selected from propylene glycol and glycerin, and more preferably propylene glycol from the viewpoints of uniformity in appearance and use as a versatile raw material.

Component (B1) is preferably a compound selected from ethanol, 1-butanol, 1-propanol, glycerin and propylene glycol, and more preferably a compound selected from ethanol and propylene glycol from the viewpoints of uniformity in appearance and use as a versatile raw material.

Component (B2) is water. Water that has been moderately purified and includes no impurities is preferably used as component (B2). Well water or industrial water can also be used. Water with small amounts of impurities or hardness components is preferably used from the viewpoint of the appearance of the sulfonate composition of the present invention. Examples of component (B2) include, for example, tap water, purified water and ion exchange water, and ion exchange water is preferable.

Component (C) is a sulfonating agent. Any compound that can be added to the double bond of the compound represented by the general formula (1) to achieve its sulfonation can be used as component (C) without any particular limitation. Component (C) is, for example, one or more compounds selected from sulfites ($M_2SO_3$ (M: cation)), hydrogen sulfites ($MHSO_3$ (M: cation)) and disulfites ($M_2S_2O_5$ (M: cation)). Component (C) is preferably an inorganic sulfate selected from hydrogen sulfites and disulfites, and more preferably an inorganic sulfate selected from disulfites. Cation (M) of component (C) may be a monovalent cation such as a sodium, potassium or ammonium ion or the like, and a divalent cation such as magnesium, calcium or the like.

Component (C) is preferably a sulfite such as ammonium sulfite, potassium sulfite, sodium sulfite or the like, a hydrogen sulfite such as ammonium hydrogen sulfite, potassium hydrogen sulfite, sodium hydrogen sulfite or the like, or a disulfite such as potassium disulfite, sodium disulfite or the like from the viewpoints of ease of handling and obtaining a high-quality and high-content sulfonate composition. Among the salts, the sodium salts or potassium salts are preferable, and the sodium salts are more preferable from the same viewpoints. Component (C) is preferably sodium disulfite or potassium disulfite and more preferably sodium disulfite from the same viewpoints.

The mixture including the sulfonate of component (A) obtained by the sulfonation reaction includes inorganic metal compounds such as unreacted component (C), a hydrolyzed product of component (C) or the like. In the removal of the insoluble component from the mixture, a deposit deposited from the mixture from which the removal of the insoluble component is made may be separated and removed. Further, the insoluble component may be deposited from the mixture from which the removal of the insoluble component is made, and the deposited deposit may be separated and removed. This deposit includes inorganic metal compound (D) such as unreacted component (C), the hydrolyzed product of component (C) or the like.

The insoluble component to be removed may be inorganic metal compound (D).

Examples of the inorganic metal compound of component (D) [hereinafter referred to as component (D)] include salts, hydroxides, oxides or the like. Component (D) may be an inorganic metal compound including no halogens.

Examples of component (D) include the inorganic sulfates, hydrolyzed products of the inorganic sulfates, neutralized products of the inorganic sulfates, neutralized products of the hydrolyzed products of the inorganic sulfates, oxides of the inorganic sulfates, oxides of the hydrolyzed products of the inorganic sulfates and oxides of the neutralized products of the inorganic sulfates or the like.

Specific examples of component (D) include one or more selected from the following (D1) to (D4):

(D1) at least one compound selected from $MHSO_3$, $M_2SO_3$ and $M_2S_2O_5$ (wherein M represents a metal cation) ;
(D2) a hydrolyzed product of compound (D1);
(D3) at least one neutralized product selected from a neutralized product of compound (D1) and a neutralized product of hydrolyzed product (D2); and
(D4) at least one oxide selected from an oxide of compound (D1), an oxide of hydrolyzed product (D2) and an oxide of neutralized product (D3).

In (D1), examples of M in the formulae include alkali metal ions, alkaline earth metal ions or the like.

Examples of $MHSO_3$ of (D1) include potassium hydrogen sulfite, sodium hydrogen sulfite or the like.

Examples of $M_2SO_3$ of (D1) include potassium sulfite, sodium sulfite or the like.

Examples of $M_2S_2O_5$ of (D1) include potassium disulfite, sodium disulfite or the like.

Examples of hydrolyzed product (D2) include potassium hydrogen sulfite, sodium hydrogen sulfite or the like when the sulfonating agent of component (D) is $M_2S_2O_5$.

Examples of neutralized product (D3) include a compound obtained by neutralizing a compound selected from compound (D1) and the hydrolyzed product thereof with a neutralizing agent.

Examples of oxide (D4) include a compound obtained by oxidizing a compound selected from compound (D1), hydrolyzed product (D2) and neutralized product (D3) with an oxidizing agent.

Next, the method for producing a sulfonate composition of the present invention is explained. The method for producing a sulfonate composition of the present invention includes reacting component (A) in the presence of component (B) with component (C) under predetermined conditions to obtain a mixture containing a sulfonate, and removing an insoluble component from the mixture.

In the sulfonation reaction of reacting component (A) with component (C), component (A) is reacted with component (C) to obtain (S) a sulfonate represented by the following general formula (2) [hereinafter referred to as component (S)]:

$$ROOC\text{-}(CH_2)_p\text{-}CH(SO_3M)\text{-}CH_2\text{-}(CH_2)_q\text{-}COOR'  \qquad (2)$$

wherein M represents a cation, and R and R' independently represent a group selected from alkyl groups with 10 carbons and alkenyl groups with 10 carbons; and p and q each represent a number of 0 or more and 18 or less, and a total of p and q is 0 or more and 18 or less.

M in the general formula (2) represents a cation, and specific examples include alkali metal ions such as a sodium ion, a potassium ion or the like and an ammonium ion.

R and R' in the general formula (2) represent the same groups as those in the aspects and suitable aspects of R and R' in the general formula (1) of component (A).

p and q each represent 0 or more, and may each represent 18 or less, 16 or less, 12 or less, 8 or less, 4 or less, 2 or less, 1 or less or 0.

The total of p and q is 0 or more, and may be 18 or less, 16 or less, 12 or less, 8 or less, 4 or less, 2 or less, 1 or less or 0.

The used amount of component (C) is preferably 1.00 mol or more and 1.20 or less by the used amount of component (C) relative to 1 mol of component (A) from the viewpoint of obtaining high-quality component (S) and the viewpoint of obtaining component (S) at a high conversion rate. The used amount of component (C) relative to 1 mol of component (A) can be selected from further more than 1.00 mol, further 1.05 mol or more, further 1.10 mol or more and further 1.13 mol or more, and further 1.17 mol or less.

In the sulfonation reaction, component (S) separately prepared can be used as a solubilizing agent (or a reaction promoting agent) or the like. As separately prepared component (S), component (S) obtained after the removal of the insoluble component or obtained by adjusting the solvent amount after the removal of the insoluble component, which will be detailed later, may be used. The used amount of component (S) added as a solubilizing agent relative to the used amount of component (A) is preferably 1 mass% or more from the viewpoint of dissolving poorly soluble component (A) in the solvents, and preferably 10 mass% or less from the viewpoint of productivity.

Unless otherwise specified, the aspects and suitable aspects described herein are common to the case where separately prepared component (S) is used and the case where it is not used.

The used amount of component (B1) in the sulfonation reaction is preferably 7 parts by mass or more, more preferably 7.5 parts by mass or more and further preferably 20 parts by mass or more, and preferably 380 parts by mass or less, more preferably 300 parts by mass or less, further preferably 216 parts by mass or less, furthermore preferably 199 parts by mass or less, furthermore preferably 100 parts by mass or less, furthermore preferably 85 parts by mass or less, furthermore preferably 75 parts by mass or less, furthermore preferably 60 parts by mass or less, furthermore preferably 55 parts by mass or less and furthermore preferably 51 parts by mass or less relative to 100 parts by mass of component (A) from the viewpoint of obtaining a high-purity sulfonate composition having a low content of inorganic metal compound (D) in the obtained sulfonate composition. The used amount of component (B1) in the sulfonation reaction preferably falls within this range both when separately prepared component (S) is used in the sulfonation reaction and when it is not.

The used amount of component (B2) in the sulfonation reaction is preferably 2.0 parts by mass or more, more preferably 2.5 parts by mass or more and further preferably 3.0 parts by mass or more, and preferably 180 parts by mass or less, more preferably 120 parts by mass or less, further preferably 50 parts by mass or less, furthermore preferably 41 parts by mass or less, furthermore preferably 39 parts by mass or less, furthermore preferably 25 parts by mass or less, furthermore preferably 18 parts by mass or less and furthermore preferably 15 parts by mass or less relative to 100 parts by mass of component (A) from the viewpoint of obtaining a high-purity sulfonate composition having a low content of inorganic metal compound (D) in the obtained sulfonate composition. The used amount of component (B2) in the sulfonation reaction preferably falls within this range both when separately prepared component (S) is used in the sulfonation reaction and when it is not.

The mass ratio of the used amount of component (B1) to the used amount of component (B2) in component (B) in the sulfonation reaction, (B1)/(B2), may be preferably 0.3 or more, more preferably 0.4 or more, further preferably 0.7 or more, furthermore preferably 0.9 or more, furthermore preferably 1.0 or more, furthermore preferably 2.0 or more and furthermore preferably 5.0 or more, and for example, 1000 or less, 100 or less, 50 or less, 30 or less, 20 or less and 10 or less from the viewpoint of obtaining a high-purity sulfonate composition having a low content of inorganic metal compound (D) in the obtained sulfonate composition. This mass ratio (B1)/(B2) preferably falls within this range both when separately prepared component (S) is used in the sulfonation reaction and when it is not.

The reaction time during which the sulfonation reaction takes place is preferably 10 hours or less from the viewpoint of obtaining high-quality component (S) and the viewpoint of achieving a high conversion rate (for example, 90% or more).

The reaction temperature in the sulfonation reaction is preferably less than 125°C and more preferably 120°C or less from the viewpoint of suppressing hydrolysis of component (S), and preferably 100°C or more, more preferably 105°C or more and further preferably 110°C or more from the viewpoint of increasing the reaction rate to shorten the reaction time.

In the sulfonation reaction, the reaction may be carried out at a normal pressure (atmospheric pressure) or under pressure depending on the boiling point of solvent (B). For example, when component (B) is propylene glycol, the reaction can be carried out even at a normal pressure as its boiling point is 188.2°C.

Further, the sulfonation reaction may be carried out under an air atmosphere or under an atmosphere of an inert gas such as a nitrogen gas or the like.

After the sulfonation reaction, concentration adjustment in the mixture obtained by the sulfonation reaction is preferably made using component (B1) and/or component (B2). For example, it is preferable that one or more solvents selected from components (B1) and (B2) be added to the mixture obtained by the sulfonation reaction, and the insoluble component be thereafter removed from this mixture.

The mass ratio of the content of component (B1) to the content of component (B2) in the mixture after the concentration adjustment, (B1)/(B2), may be adjusted to preferably 0.3 or more, more preferably 0.4 or more, further preferably 0.7 or more, furthermore preferably 0.9 or more and furthermore preferably 1.0 or more, and for example, 1000 or less, 100 or less, 50 or less, 30 or less, 20 or less and 10 or less from the viewpoint of obtaining a high-purity sulfonate composition having a low content of inorganic metal compound (D) in the obtained sulfonate composition. This mass ratio (B1)/(B2) preferably falls within this range both when separately prepared component (S) is used in the sulfonation reaction and when it is not.

When component (B1) is mixed to the mixture obtained by the sulfonation reaction, component (B1) can be mixed such that the content of component (B1) in the composition after component (B1) is mixed to the mixture obtained by the sulfonation reaction is preferably 5 parts by mass or more, more preferably 10 parts by mass or more and further preferably 15 parts by mass or more, and preferably 380 parts by mass or less, more preferably 300 parts by mass or less, further preferably 200 parts by mass or less and furthermore preferably 60 parts by mass or less with respect to 100 parts by mass of component (A) before the sulfonation reaction from the viewpoints of ease of handling in terms of physical properties and operability in a subsequent step. If the content of component (B1) in the mixture obtained by the sulfonation reaction falls within the above range, a subsequent step may be carried out without mixing component (B1).

When component (B2) is mixed to the mixture obtained by the sulfonation reaction, component (B2) can be mixed such that the content of component (B2) in the composition after component (B2) is mixed to the mixture obtained by the sulfonation reaction is preferably 5 parts by mass or more, more preferably 15 parts by mass or more and further preferably 25 parts by mass or more, and preferably 180 parts by mass or less, more preferably 120 parts by mass or less, further preferably 80 parts by mass or less and furthermore preferably 50 parts by mass or less with respect to 100 parts by mass of component (A) before the sulfonation reaction from the viewpoints of ease of handling in terms of physical properties and operability in a subsequent step. If the content of component (B2) in the mixture obtained by the sulfonation reaction falls within the above range, a subsequent step may be carried out without mixing component (B2).

When component (B1) is mixed to the mixture obtained by the sulfonation reaction, component (B1) can be mixed to the mixture obtained by the sulfonation reaction in an amount of, for example, 1 part by mass or more, 1.5 parts by mass or more, 10 parts by mass or more and 20 parts by mass or more, and 350 parts by mass or less, 320 parts by mass or less, 300 parts by mass or less, 250 parts by mass or less, 200 parts by mass or less, 150 parts by mass or less, 100 parts by mass or less, 75 parts by mass or more and 50 parts by mass or less with respect to 100 parts by mass of component (A) before the sulfonation reaction from the viewpoints of ease of handling in terms of physical properties and operability in a subsequent step.

When component (B2) is mixed to the mixture obtained by the sulfonation reaction, component (B2) can be mixed to the mixture obtained by the sulfonation reaction in an amount of, for example, 1 part by mass or more, 5 parts by mass or more, 15 parts by mass or more and 20 parts by mass or more, and 150 parts by mass or less, 100 parts by mass or less, 50 parts by mass or less, 40 parts by mass or less, 35 parts by mass or less and 30 parts by mass or less with respect to 100 parts by mass of component (A) before the sulfonation reaction from the viewpoints of ease of handling in terms of physical properties and operability in a subsequent step.

In the removal of the insoluble component from the mixture obtained by the sulfonation reaction, the insoluble component such as component (D) or the like is removed from the mixture. In the removal of the insoluble component, for example, the insoluble component deposited from the mixture is separated and removed by filtration, centrifugation or the like. Unreacted component (C) and/or the hydrolyzed product thereof in the mixture from which the removal of the insoluble component is made may be neutralized and/or oxidized before the removal of the insoluble component. When the neutralization reaction and the oxidation reaction are carried out, the oxidation reaction may be carried out after the neutralization reaction or the neutralization reaction may be carried out after the oxidation reaction.

The reaction temperature in the neutralization reaction and the oxidation reaction is preferably 60°C or less and more preferably 55°C or less from the viewpoint of suppressing hydrolysis of component (S), and preferably 20°C or more from the viewpoint of operability.

Note that the reaction temperature in the neutralization reaction and the oxidation reaction refers to the temperature of the mixture obtained by the sulfonation reaction during the neutralization reaction and the oxidation reaction. As neutralization reactions and oxidation reactions are exothermic, the mixture can be cooled as necessary to perform the operations in the above temperature range, thereby suppressing hydrolysis of component (S).

While a neutralizing agent used in the neutralization reaction is not particularly limited, alkali metal carbonates such as sodium carbonate, potassium carbonate or the like, alkali metal hydrogen carbonates such as sodium hydrogen carbonate, potassium hydrogen carbonate or the like, hydroxides of alkali metals such as sodium hydroxide, potassium hydroxide or the like can be used, and they can be used in the form of a powder or an aqueous solution. An aqueous sodium carbonate solution, an aqueous sodium hydrogen carbonate or sodium hydroxide solution is preferable from the viewpoints of ease of handling and obtaining a high-quality sulfonate. The concentration of an aqueous solution of the neutralizing agent is preferably 1 mass% or more and 48 mass% or less. When the neutralizing agent is an aqueous sodium carbonate solution or an aqueous sodium hydrogen carbonate solution, an aqueous solution containing sodium carbonate or sodium hydrogen carbonate at a concentration of 2 mass% or more and 22 mass% or less is further preferable. Further, when the neutralizing agent is an aqueous sodium hydroxide solution, an aqueous solution containing sodium hydroxide at a concentration of 2 mass% or more and 35 mass% or less is further preferable.

While an oxidizing agent used in the oxidation reaction is not particularly limited, alkali metal or alkaline earth metal peroxides, alkali metal or alkaline earth metal salts of hypochlorous acid, potassium permanganate, hydrogen peroxide or the like are preferable, and hydrogen peroxide is more preferable from the viewpoints of ease of handling, the efficiency of the oxidation treatment and obtaining a high-quality sulfonate. As the oxidizing agent, hydrogen peroxide water obtained by dissolving hydrogen peroxide in water can be used.

The content of component (B1) in the mixture from which the removal of the insoluble component is made is preferably 5 parts by mass or more, more preferably 7 parts by mass or more, further preferably 7.5 parts by mass or more, furthermore preferably 8 parts by mass or more, furthermore preferably 10 parts by mass or more and furthermore preferably 15 parts by mass or more, and preferably 380 parts by mass or less, more preferably 300 parts by mass or less, further preferably 216 parts by mass or less, furthermore preferably 200 parts by mass or less, furthermore preferably 199 parts by mass or less, furthermore preferably 100 parts by mass or less, furthermore preferably 85 parts by mass or less, furthermore preferably 75 parts by mass or less, furthermore preferably 65 parts by mass or less, furthermore preferably 60 parts by mass or less and furthermore preferably 55 parts by mass or less with respect to 100 parts by mass of component (A) before the sulfonation reaction from the viewpoint of obtaining a high-purity sulfonate composition having a low content of inorganic metal compounds in the sulfonate composition.

The content of component (B1) in the mixture from which the removal of the insoluble component is made is preferably 5 mass% or more and more preferably 7 mass% or more, and preferably 65 mass% or less, more preferably 60 mass% or less, further preferably 40 mass% or less, furthermore preferably 30 mass% or less and furthermore preferably 20 mass% or less from the same viewpoint.

In either case, the content of component (B1) in the mixture from which the removal of the insoluble component is made preferably falls within the above range both when separately prepared component (S) is used in the sulfonation reaction and when it is not.

Note that, as the contents of components (B1) and (B2) in the mixture from which the removal of the insoluble component is made, calculated values determined on the basis of a measured value in the mixture, a feed amount of component (A) for the sulfonation reaction and a measured value of the content of each component in the mixture after the removal of the insoluble component may be employed (the same applies hereinafter).

The content of component (B2) in the mixture from which the removal of the insoluble component is made is preferably 5 parts by mass or more, more preferably 7 parts by mass or more, further preferably 15 parts by mass or more and furthermore preferably 25 parts by mass or more, and preferably 180 parts by mass or less, more preferably 120 parts by mass or less, further preferably 80 parts by mass or less, furthermore preferably 45 parts by mass or less, furthermore preferably 40 parts by mass or less, furthermore preferably 37 parts by mass or less, furthermore preferably 35 parts by mass or less and furthermore preferably 30 parts by mass or less with respect to 100 parts by mass of component (A) before the sulfonation reaction from the viewpoint of obtaining a high-purity sulfonate composition having a low content of inorganic metal compounds in the sulfonate composition.

The content of component (B2) in the mixture from which the removal of the insoluble component is made is preferably 3 mass% or more and more preferably 5 mass% or more, and preferably 40 mass% or less, more preferably 35 mass% or less, further preferably 30 mass% or less, furthermore preferably 26 mass% or less, furthermore preferably 25 mass% or less, furthermore preferably 22 mass% or less and furthermore preferably 20 mass% or less from the same viewpoint.

In either case, the content of component (B2) in the mixture from which the removal of the insoluble component is made preferably falls within the above range both when separately prepared component (S) is used in the sulfonation reaction and when it is not.

The mass ratio of the content of component (B1) to the total content of components (S), (B1) and (B2) in the mixture from which the removal of the insoluble component is made, (B1)/(S + B1 + B2), is 0.05 or more, preferably 0.09 or more and more preferably 0.10 or more from the viewpoint of the removal of inorganic metal compounds, and 0.65 or less, preferably 0.59 or less, more preferably 0.55 or less, further preferably 0.50 or less, furthermore preferably 0.40 or less and furthermore preferably 0.35 or less from the viewpoint of obtaining a sulfonate composition of a high degree of purity and/or a higher concentration.

The mass ratio of the content of component (B2) to the total content of components (S), (B1) and (B2) in the mixture from which the removal of the insoluble component is made, (B2)/(S + B1 + B2), is 0.03 or more and preferably 0.10 or more from the viewpoint of the removal of inorganic metal compounds, and 0.26 or less, preferably 0.25 or less, more preferably 0.23 or less, further preferably 0.20 or less and furthermore preferably 0.18 or less from the viewpoint of obtaining a sulfonate composition of a high degree of purity and/or a higher concentration.

The mass ratio of the content of component (B1) to the content of component (B2) in the mixture from which the removal of the insoluble component is made, (B1) / (B2), is preferably 0.3 or more, and for example, can be selected from 0.6 or more, 0.80 or more, 0.82 or more, 0.85 or more and 0.9 or more, and can be selected from 1000 or less, 100 or less, 50 or less, 30 or less, 20 or less, 10 or less, 5 or less, 2.5 or less and 2 or less from the viewpoint of obtaining a high-purity sulfonate composition having a low content of inorganic metal compounds in the sulfonate composition. From the viewpoint of depositing a large amount of inorganic metal compounds such as inorganic salts or the like in the mixture, it is sufficient for the mixture to contain only a small amount of component (B2), and thus, the upper limit of this mass ratio (B1)/(B2) can be arbitrarily selected considering the amount of component (S) in the mixture and the solubility of component (S) in component (B1) .

If the contents of components (B1) and (B2) in the mixture from which the removal of the insoluble component is made are as above, the amount of inorganic metal compounds dissolving in the mixture can be reduced while dissolving component (S) in the mixture. If the insoluble component is removed from this mixture, a mixture including a high concentration of component (S) and having a low content of inorganic metal compounds can be obtained.

The preferable aspects of the content of (B1), the content of (B2) and the mass ratio of the content of component (B1) to the content of component (B2), (B1)/(B2), in the mixture from which the removal of the insoluble component is made are as described above both when separately prepared component (S) is used in the sulfonation reaction and when it is not.

The used amounts of components (B1) and (B2) in the sulfonation reaction may be determined such that the contents of components (B1) and (B2) fall within the above parts by mass (or content) ranges. Further, one or more solvents selected from components (B1) and (B2) can be added to the mixture before the removal of the insoluble component is made to adjust the contents of components (B1) and (B2) in the mixture, and the insoluble component can be removed from such a mixture. The contents of components (B1) and (B2) in the mixture from which the removal of the insoluble component is made can be adjusted during the sulfonation reaction or after the sulfonation reaction but before the removal of the insoluble component.

The viscosity at 20°C of the mixture from which the removal of the insoluble component is made is preferably 600 mPa·s or less, more preferably 500 mPa·s or less, further preferably 470 mPa·s or less and furthermore preferably 450 mPa·s or less, and may be, for example, 0.1 mPa·s or more, 1 mPa·s or more and 10 mPa·s or more from the viewpoints of ease of handling in terms of physical properties and operability in a subsequent step. The viscosity of the mixture can be measured at 20°C using a B-type rotational viscometer.

In the removal of the insoluble component, for example, the mixture from which the removal of the insoluble component is made may be cooled to 50°C or less to deposit component (D) from the mixture, and component (D) may be separated and removed by a publicly-known separation method. Further, the insoluble component may be deposited from the mixture from which the removal of the insoluble component is made, and the deposit from the mixture such as component (D) or the like may be separated and removed by a publicly-known separation method.

Note that azeotropic distillation is preferably not carried out in the removal of the insoluble component from the viewpoint of obtaining a high-purity sulfonate.

Examples of the above separation method include treatment with an adsorption membrane or an adsorbent, addition of a flocculating agent, centrifugation, leaving the mixture to stand, decantation, filtration or the like. The filtration may be gravity filtration, pressure filtration, filtration under reduced pressure or the like. Further, in the removal of the insoluble component, a filter aid or a flocculating agent may be used together as necessary. Examples of the filter aid are not particularly limited, and diatomaceous earth, silica, glass fiber or activated carbon for industrial use or the like can be used.

When a filter aid is used, the added amount of the filter aid is preferably 0.01 parts by mass or more and more preferably 1 part by mass or more, and preferably 20 parts by mass or less and more preferably 10 parts by mass or less per 100 parts by mass of the mixture from which the removal of the insoluble component is made from the viewpoint of removing inorganic metal compounds and the viewpoint of the amount of a liquid contained in the filter aid.

Further, the particle size of component (D) captured during the insoluble component removal operation may be, for example, 10 um or less and 5 um or less, and 0.1 um or more and 1 um or more from the viewpoints of the rate of removal of the insoluble component and operability in a subsequent step. This particle size is a value guaranteed as the captured particle size of the filter aid and filter media used. In other words, this particle size is the particle size of particles captured by the opening of the filter means.

The insoluble component is preferably removed such that the content of sodium sulfate in the mixture after the removal of the insoluble component is preferably 1.2 mass% or less, more preferably 0.8 mass% or less, further preferably 0.7 mass% or less and furthermore preferably 0.5 mass% or less from the viewpoints of stability in the appearance of the sulfonate composition, and suppression of turbidity or precipitate formation and low-temperature stability when the sulfonate composition is used for the formulation of any product together with other components.

The method for producing a sulfonate composition of the present invention can produce a sulfonate composition that can be utilized as-is as a product. This sulfonate composition may be utilized for secondary products. The method for producing a sulfonate composition of the present invention may be a method in which component (B1) is not added after the production of the sulfonate, i.e., after the removal of the insoluble component, and before the sulfonate composition is utilized for secondary products.

Note that, in the method for producing a sulfonate composition of the present invention, a solvent including one or more selected from components (B1) and (B2) may be added after the removal of the insoluble component to adjust the solvent amount in the sulfonate composition such that the sulfonate composition contains component (S) or the like at a desired concentration.

The method for producing a sulfonate composition of the present invention can produce the sulfonate composition including component (S).

The content of component (B1) in the sulfonate composition of the present invention is preferably 5 mass% or more, more preferably 10 mass% or more and further preferably 12 mass% or more, and preferably 65 mass% or less, more preferably 58 mass% or less, further preferably 35 mass% or less and furthermore preferably 30 mass% or less from the viewpoint of obtaining a sulfonate composition having a low content of inorganic metal compounds and the viewpoint of suppressing turbidity or precipitation caused by the deposition of inorganic metal compounds. It is preferable that the content of (B1) fall within this range and the content of (B2) fall within the following range.

The content of component (B2) in the sulfonate composition of the present invention is preferably 2 mass% or more, more preferably 3 mass% or more, further preferably 4 mass% or more and furthermore preferably 5 mass% or more, and preferably 30 mass% or less, more preferably 25 mass% or less and further preferably 20 mass% or less from the viewpoint of obtaining a sulfonate composition having a low content of inorganic metal compounds and the viewpoint of suppressing turbidity or precipitation caused by the deposition of inorganic metal compounds.

The contents of components (B1) and (B2) in the sulfonate composition of the present invention may be the contents after the removal of the insoluble component or the contents after the adjustment of the solvent amount after the removal of the insoluble components.

The sulfonate composition of the present invention can contain component (S) in an amount of preferably 50 mass% or more and more preferably 55 mass% or more, and preferably 70 mass% or less and more preferably 68 mass% or less from the viewpoints of uniformity in appearance and production efficiency.

Further, the sulfonate composition of the present invention can contain component (S) in an amount of preferably 19 mass% or more, and preferably 80 mass% or less, preferably 70 mass% or less and more preferably 68 mass% or less from the viewpoints of uniformity in appearance and operability.

The sulfonate composition of the present invention can contain component (B) in an amount of preferably 20 mass% or more and more preferably 25 mass% or more, and preferably 45 mass% or less from the viewpoints of production efficiency, uniformity in appearance and operability.

Further, the sulfonate composition of the present invention can contain component (B) in an amount of preferably 45 mass% or more, and preferably 80 mass% or less from the viewpoints of uniformity in appearance and operability.

The content of component (D) in the sulfonate composition of the present invention is, relative to 100 parts by mass of the content of component (S), preferably less than 1.6 parts by mass, more preferably 1.5 parts by mass or less and further preferably 1.0 parts by mass or less from the viewpoint of the degree of purity of component (S), and may be, for example, 0.10 parts by mass or more and further 0.15 parts by mass or more from the viewpoint of productivity.

The sulfonate composition of the present invention can contain components other than components (S) and (B). For example, the composition can contain maleic acid or fumaric acid diesters or monoesters, 2-propylheptanol, 4-methyl 2-propylhexanol, 5-methyl 2-propylhexanol, sulfosuccinic acid monoester salts, maleic acid or fumaric acid salts and others, as well as additives or adjusters such as pH buffers, antiseptics or the like.

The sulfonate composition of the present invention can be used as a raw material for products such as, for example, detergents, solubilizing agents, treatment agents, modifiers, wetting agents, penetrating agents, agrochemicals, paints, cosmetics and emulsifiers or the like.

An example of an aspect of the method for producing a sulfonate composition of the present invention is a method for producing a sulfonate composition including, reacting (A) a compound represented by the general formula (1) in the presence of one or more solvents selected from (B) (B1) an organic solvent and (B2) water with (C) a sulfonating agent to obtain a mixture containing (S) a sulfonate represented by the following general formula (2), and removing an insoluble component from the mixture,

$$\text{ROOC-}(CH_2)_n\text{-CH=CH-}(CH_2)_m\text{-COOR'} \qquad (1)$$

wherein R and R' independently represent a group selected from alkyl groups with 10 carbons and alkenyl groups with 10 carbons; and n and m each represent a number of 0 or more and 18 or less, and a total of n and m is 0 or more and 18 or less,

$$\text{ROOC-}(CH_2)_p\text{-CH(SO}_3\text{M)-CH}_2\text{-}(CH_2)_q\text{-COOR'} \qquad (2)$$

wherein M represents a cation, and R and R' independently represent a group selected from alkyl groups with 10 carbons and alkenyl groups with 10 carbons; and p and q each represent a number of 0 or more and 18 or less, and a total of p and q is 0 or more and 18 or less,

wherein the mixture from which the removal of the insoluble component is made contains components (S), (B1) and (B2), wherein a mass ratio of the content of component (B1) to a total content of components (S), (B1) and (B2), (B1)/(S + B1 + B2), is 0.05 or more, and 0.65 or less and preferably 0.59 or less, a mass ratio of the content of component (B2) to the total content of components (S), (B1) and (B2), (B2)/(S + B1 + B2), is 0.03 or more, and 0.26 or less and preferably 0.20 or less, and preferably, a mass ratio of the content of component (B1) to the content of component (B2), (B1)/(B2), is 0.80 or more. This method for producing a sulfonate composition may include depositing the insoluble component from the mixture containing sulfonate (S) represented by the general formula (2), and removing the component deposited from the mixture.

The insoluble component may be deposited from the mixture by carrying out one of or a combination of two or more of methods selected from an oxidation reaction of the component in the mixture, a neutralization reaction of the component in the mixture and cooling of the mixture.

Further, the method for producing a sulfonate composition of the present invention may include, after reacting component (A) with component (C) to obtain the mixture containing component (S), removing the insoluble component without heating the mixture (for example, raising the temperature to 80°C or more) from the viewpoint of obtaining high-quality component (S). Further, the method preferably includes, after reacting component (A) with component (C) to obtain the mixture containing component (S), removing the insoluble component without carrying out azeotropic distillation from the viewpoint of obtaining a high-purity sulfonate.

<Aspects of the present invention>

The following aspects of the present invention are described by way of example. Also in the following aspects, a compound represented by the general formula (1) is referred to as component (A), an organic solvent as component (B1), water as component (B2), one or more solvents selected from components (B1) and (B2) as component (B), a sulfonating agent as component (C) and a sulfonate represented by the general formula (2) as component (S).

<1> A method for producing a sulfonate composition including, reacting (A) a compound represented by the following general formula (1) in the presence of one or more solvents selected from (B) (B1) an organic solvent and (B2) water with (C) a sulfonating agent to obtain a mixture containing (S) a sulfonate represented by the following general formula (2), and removing an insoluble component from the mixture,

$$ROOC\text{-}(CH_2)_n\text{-}CH\text{=}CH\text{-}(CH_2)_m\text{-}COOR' \qquad (1)$$

wherein R and R' independently represent a group selected from alkyl groups with 10 carbons and alkenyl groups with 10 carbons; and n and m each represent a number of 0 or more and 18 or less, and a total of n and m is 0 or more and 18 or less,

$$ROOC\text{-}(CH_2)_p\text{-}CH(SO_3M)\text{-}CH_2\text{-}(CH_2)_q\text{-}COOR' \qquad (2)$$

wherein M represents a cation, and R and R' independently represent a group selected from alkyl groups with 10 carbons and alkenyl groups with 10 carbons; and p and q each represent a number of 0 or more and 18 or less, and a total of p and q is 0 or more and 18 or less,
wherein the mixture from which the removal of the insoluble component is made contains components (S), (B1) and (B2), wherein a mass ratio of the content of component (B1) to a total content of components (S), (B1) and (B2), (B1)/(S + B1 + B2), is 0.05 or more and 0.65 or less, and a mass ratio of the content of component (B2) to the total content of components (S), (B1) and (B2), (B2)/(S + B1 + B2), is 0.03 or more and 0.26 or less.

<2> The method for producing a sulfonate composition according to <1>, wherein component (A) is a compound of the general formula (1) in which R and R' independently represent a group selected from 2-propylheptyl group, 4-methyl 2-propylhexyl group and 5-methyl 2-propylhexyl group.
<3> The method for producing a sulfonate composition according to <1> or <2>, wherein one or more solvents selected from components (B1) and (B2) are added to the mixture, and the insoluble component is thereafter removed from the mixture.
<4> The method for producing a sulfonate composition according to any of <1> to <3>, wherein in the sulfonate composition, the content of component (B1) is 5 mass% or more and 65 mass% or less, and the content of component (B2) is 3 mass% or more and 25 mass% or less.
<5> The method for producing a sulfonate composition according to any of <1> to <4>, wherein component (B1) is at least one selected from a monohydric alcohol and a polyhydric alcohol.
<6> The method for producing a sulfonate composition according to any of <1> to <5>, wherein component (C) is at least one compound selected from $MHSO_3$, $M_2SO_3$ and $M_2S_2O_5$, wherein M represents a cation.

<7> The method for producing a sulfonate composition according to any of <1> to <6>, wherein component (C) is used at a ratio of 1.00 mol or more and 1.20 mol or less relative to 1 mol of component (A).

<8> The method for producing a sulfonate composition according to any of <1> to <7>, wherein component (A) is reacted with component (C) at a reaction temperature of 100°C or more and less than 125°C and at a normal pressure or under pressure.

<9> The method for producing a sulfonate composition according to any of <1> to <8>, wherein an unreacted component (C) in the mixture is neutralized and/or oxidized before removing the insoluble component.

<10> The method for producing a sulfonate composition according to any of <1> to <9>, wherein a sulfonate obtained by reacting component (A) with component (C) is prepared separately (hereinafter referred to as separately prepared component (S)), and component (A) is reacted with component (C) under the coexistence of the sulfonate.

<11> The method for producing a sulfonate composition according to any of <1> to <10>, wherein R and R' in the general formula (1) independently represent a group selected from branched-chain alkyl groups with 10 carbons and branched-chain alkenyl groups with 10 carbons, and further a branched-chain alkyl group with 10 carbons.

<12> The method for producing a sulfonate composition according to any of <1> to <10>, wherein R and R' in the general formula (1) independently represent a group selected from n-decyl group, iso-decyl group, 2-propylheptyl group, 4-methyl 2-propylhexyl group and 5-methyl 2-propylhexyl group.

<13> The method for producing a sulfonate composition according to any of <1> to <10>, wherein R and R' in the general formula (1) independently represent a group selected from n-decenyl group and iso-decenyl group.

<14> The method for producing a sulfonate composition according to any of <1> to <13>, wherein one or two or more types of components (A) are used.

<15> The method for producing a sulfonate composition according to any of <1> to <14>, wherein component (A) is a compound for which one of or both R and R' represent 2-propylheptyl group.

<16> The method for producing a sulfonate composition according to any of <1> to <15>, wherein n and m in the general formula (1) each represent 0 or more, and 18 or less, 16 or less, 12 or less, 8 or less, 4 or less, 2 or less, 1 or less or 0.

<17> The method for producing a sulfonate composition according to any of <1> to <16>, wherein the total of n and m in the general formula (1) is 0 or more, and 18 or less, 16 or less, 12 or less, 8 or less, 4 or less, 2 or less, 1 or less or 0.

<18> The method for producing a sulfonate composition according to any of <5> to <17>, wherein the monohydric alcohol has 1 or more and 12 or less carbons, and is further one or more compounds selected from methanol, ethanol, 1-propanol, 1-butanol, 1-pentanol, 1-hexanol, 1-octanol, 1-nonanol, 2-nonanol, 5-nonanol, 1-decanol, 1-dodecanol, 3,5,5-trimethylhexan-1-ol, 2,6-dimethyl-4-heptanol, 2-propylheptanol, 4-methyl 2-propylhexanol and 5-methyl 2-propylhexanol, further one or more compounds selected from ethanol, 1-butanol and 1-propanol, and further ethanol.

<19> The method for producing a sulfonate composition according to any of <5> to <17>, wherein the polyhydric alcohol has 2 or more and 12 or less carbons and is dihydric or more and hexahydric or less.

<20> The method for producing a sulfonate composition according to any of <5> to <17> and <19>, wherein the polyhydric alcohol is one or more compounds selected from ethylene glycol, propylene glycol, 1,3-butanediol, 1,4-butanediol, 1,5-pentanediol, 1,6-hexanediol, 1,10-decanediol, diethylene glycol, triethylene glycol, tetraethylene glycol, dipropylene glycol, tripropylene glycol, glycerin, xylitol and sorbitol, further one or more compounds selected from propylene glycol and glycerin, and further propylene glycol.

<21> The method for producing a sulfonate composition according to any of <1> to <20>, wherein component (B1) is one or more compounds selected from ethanol, 1-butanol, 1-propanol, glycerin and propylene glycol, and further one or more compounds selected from ethanol and propylene glycol.

<22> The method for producing a sulfonate composition according to any of <1> to <21>, wherein component (B2) is any of tap water, purified water and ion exchange water.

<23> The method for producing a sulfonate composition according to any of <1> to <22>, wherein component (C) is one or more compounds selected from sulfites, hydrogen sulfites and disulfites, and further, the sulfites are one or more sulfites selected from ammonium sulfite, potassium sulfite and sodium sulfite, the hydrogen sulfites are one or more hydrogen sulfites selected from ammonium hydrogen sulfite, potassium hydrogen sulfite and sodium hydrogen sulfite, and the disulfites are one or more disulfites selected from potassium disulfite and sodium disulfite.

<24> The method for producing a sulfonate composition according to any of <1> to <23>, wherein component (C) is sodium disulfite or potassium disulfite, and further sodium disulfite.

<25> The method for producing a sulfonate composition according to any of <1> to <24>, wherein R and R' in the general formula (2) independently represent a group selected from branched-chain alkyl groups with 10 carbons and branched-chain alkenyl groups with 10 carbons, and further a branched-chain alkyl group with 10 carbons.

<26> The method for producing a sulfonate composition according to any of <1> to <24>, wherein R and R' in the general formula (2) independently represent a group selected from n-decyl group, iso-decyl group, 2-propylheptyl group, 4-methyl 2-propylhexyl group and 5-methyl 2-propylhexyl group.

<27> The method for producing a sulfonate composition according to any of <1> to <24>, wherein R and R' in the general formula (2) independently represent a group selected from n-decenyl group and iso-decenyl group.

<28> The method for producing a sulfonate composition according to any of <1> to <24>, wherein R and R' in the general formula (2) independently represent a group selected from 2-propylheptyl group, 4-methyl 2-propylhexyl group and 5-methyl 2-propylhexyl group.

<29> The method for producing a sulfonate composition according to any of <1> to <28>, wherein p and q in the general formula (2) each represent 0 or more, and 18 or less, 16 or less, 12 or less, 8 or less, 4 or less, 2 or less, 1 or less or 0.

<30> The method for producing a sulfonate composition according to any of <1> to <29>, wherein the total of p and q in the general formula (2) is 0 or more, and 18 or less, 16 or less, 12 or less, 8 or less, 4 or less, 2 or less, 1 or less or 0.

<31> The method for producing a sulfonate composition according to any of <1> to <30>, wherein a used amount of component (C) relative to 1 mol of component (A) is selected from any of more than 1.00 mol, 1.05 mol or more, 1.10 mol or more and 1.13 mol or more, and is 1.17 mol or less.

<32> The method for producing a sulfonate composition according to any of <1> to <31>, wherein, in the sulfonation reaction, component (B1) is used in an amount of preferably 7 parts by mass or more, more preferably 7.5 parts by mass or more and further preferably 20 parts by mass or more, and preferably 380 parts by mass or less, more preferably 300 parts by mass or less, further preferably 216 parts by mass or less, furthermore preferably 199 parts by mass or less, furthermore preferably 100 parts by mass or less, furthermore preferably 85 parts by mass or less, furthermore preferably 75 parts by mass or less, furthermore preferably 60 parts by mass or less, furthermore preferably 55 parts by mass or less and furthermore preferably 51 parts by mass or less relative to 100 parts by mass of component (A).

<33> The method for producing a sulfonate composition according to any of <1> to <32>, wherein, in the sulfonation reaction, component (B2) is used in an amount of preferably 2.0 parts by mass or more, more preferably 2.5 parts by mass or more and further preferably 3.0 parts by mass or more, and preferably 180 parts by mass or less, more preferably 120 parts by mass or less, further preferably 50 parts by mass or less, furthermore preferably 41 parts by mass or less, furthermore preferably 39 parts by mass or less, furthermore preferably 25 parts by mass or less, furthermore preferably 18 parts by mass or less and furthermore preferably 15 parts by mass or less relative to 100 parts by mass of component (A).

<34> The method for producing a sulfonate composition according to any of <1> to <33>, wherein, in the sulfonation reaction, a mass ratio of a used amount of component (B1) to a used amount of component (B2), (B1)/(B2), is preferably 0.3 or more, more preferably 0.4 or more, further preferably 0.7 or more, furthermore preferably 0.9 or more, furthermore preferably 1.0 or more, furthermore preferably 2.0 or more and furthermore preferably 5.0 or more.

<35> The method for producing a sulfonate composition according to any of <1> to <34>, wherein, after the sulfonation reaction, concentration adjustment in the mixture obtained by the sulfonation reaction is made using component (B1) and/or component (B2).

<36> The method for producing a sulfonate composition according to <35>, wherein a mass ratio of the content of component (B1) to the content of component (B2) in the mixture after the concentration adjustment, (B1)/(B2), is preferably 0.3 or more, more preferably 0.4 or more, further preferably 0.7 or more, furthermore preferably 0.9 or more and furthermore preferably 1.0 or more.

<37> The method for producing a sulfonate composition according to any of <1> to <36>, wherein the content of component (B1) in the mixture from which the removal of the insoluble component is made is preferably 5 parts by mass or more, more preferably 7 parts by mass or more, further preferably 7.5 parts by mass or more, furthermore preferably 8 parts by mass or more, furthermore preferably 10 parts by mass or more and furthermore preferably 15 parts by mass or more, and preferably 380 parts by mass or less, more preferably 300 parts by mass or less, further preferably 216 parts by mass or less, furthermore preferably 200 parts by mass or less, furthermore preferably 199 parts by mass or less, furthermore preferably 100 parts by mass or less, furthermore preferably 85 parts by mass or less, furthermore preferably 75 parts by mass or less, furthermore preferably 65 parts by mass or less, furthermore preferably 60 parts by mass or less and furthermore preferably 55 parts by mass or less with respect to 100 parts by mass of component (A) before the sulfonation reaction.

<38> The method for producing a sulfonate composition according to any of <1> to <37>, wherein the content of component (B1) in the mixture from which the removal of the insoluble component is made is preferably 5 mass% or more and more preferably 7 mass% or more, and preferably 65 mass% or less, more preferably 60 mass% or less, further preferably 40 mass% or less, furthermore preferably 30 mass% or less and furthermore preferably 20 mass% or less.

<39> The method for producing a sulfonate composition according to any of <1> to <38>, wherein the content of component (B2) in the mixture from which the removal of the insoluble component is made is preferably 5 parts by mass or more, more preferably 7 parts by mass or more, further preferably 15 parts by mass or more and furthermore preferably 25 parts by mass or more, and preferably 180 parts by mass or less, more preferably 120 parts by mass or less, further preferably 80 parts by mass or less, furthermore preferably 45 parts by mass or less, furthermore preferably 40 parts by mass or less, furthermore preferably 37 parts by mass or less, furthermore preferably 35 parts by mass or less and furthermore preferably 30 parts by mass or less with respect to 100 parts by mass of component (A) before the sulfonation reaction.

<40> The method for producing a sulfonate composition according to any of <1> to <39>, wherein the content of component (B2) in the mixture from which the removal of the insoluble component is made is preferably 3 mass% or more and more preferably 5 mass% or more, and preferably 40 mass% or less, more preferably 35 mass% or less, further preferably 30 mass% or less, furthermore preferably 26 mass% or less, furthermore preferably 25 mass% or less, furthermore preferably 22 mass% or less and furthermore preferably 20 mass% or less.

<41> The method for producing a sulfonate composition according to any of <1> to <40>, wherein the mass ratio of the content of component (B1) to the total content of components (S), (B1) and (B2) in the mixture from which the removal of the insoluble component is made, (B1)/(S + B1 + B2), is 0.05 or more, preferably 0.09 or more and more preferably 0.10 or more, and 0.65 or less, preferably 0.59 or less, more preferably 0.55 or less, further preferably 0.50 or less, furthermore preferably 0.40 or less and furthermore preferably 0.35 or less.

<42> The method for producing a sulfonate composition according to any of <1> to <41>, wherein the mass ratio of the content of component (B2) to the total content of components (S), (B1) and (B2) in the mixture from which the removal of the insoluble component is made, (B2)/(S + B1 + B2), is 0.03 or more and preferably 0.10 or more, and 0.26 or less, preferably 0.25 or less, more preferably 0.23 or less, further preferably 0.20 or less and furthermore preferably 0.18 or less.

<43> The method for producing a sulfonate composition according to any of <1> to <42>, wherein a mass ratio of the content of component (B1) to the content of component (B2) in the mixture from which the removal of the insoluble component is made, (B1)/(B2), is preferably 0.3 or more, for example, 0.6 or more, 0.80 or more, 0.82 or more, 0.85 or more and 0.9 or more, and 1000 or less, 100 or less, 50 or less, 30 or less, 20 or less, 10 or less, 5 or less, 2.5 or less and 2 or less.

<44> The method for producing a sulfonate composition according to any of <1> to <43>, wherein the insoluble component is removed from the mixture by one or more separation methods selected from an adsorption membrane or an adsorbent, addition of a flocculating agent, centrifugation, leaving the mixture to stand, decantation and filtration.
<45> The method for producing a sulfonate composition according to <44>, wherein the filtration is gravity filtration, pressure filtration or filtration under reduced pressure.

<46> The method for producing a sulfonate composition according to <44> or <45>, wherein one or more selected from a filter aid and a flocculating agent are used to remove the insoluble component from the mixture, and further, the filter aid is one or more filter aids selected from diatomaceous earth, silica and glass fiber.
<47> The method for producing a sulfonate composition according to <46>, wherein an added amount of the filter aid is preferably 0.01 parts by mass or more and more preferably 1 part by mass or more, and preferably 20 parts by mass or less and more preferably 10 parts by mass or less per 100 parts by mass of the mixture from which the removal of the insoluble component is made.

<48> The method for producing a sulfonate composition according to any of <1> to <47>, wherein the content of sodium sulfate in the mixture after the removal of the insoluble component is preferably 1.2 mass% or less, more preferably 0.8 mass% or less, further preferably 0.7 mass% or less and furthermore preferably 0.5 mass% or less.

<49> The method for producing a sulfonate composition according to any of <1> to <48>, wherein the content of component (B1) in the obtained sulfonate composition is preferably 5 mass% or more, more preferably 10 mass% or more and further preferably 12 mass% or more, and preferably 65 mass% or less, more preferably 58 mass% or less, further preferably 35 mass% or less and furthermore preferably 30 mass% or less.

<50> The method for producing a sulfonate composition according to any of <1> to <49>, wherein the content of component (B2) in the obtained sulfonate composition is preferably 2 mass% or more, more preferably 3 mass% or more, further preferably 4 mass% or more and furthermore preferably 5 mass% or more, and preferably 30 mass% or less, more preferably 25 mass% or less and further preferably 20 mass% or less.

<51> The method for producing a sulfonate composition according to <1> to <50>, wherein the obtained sulfonate composition contains component (S) in an amount of preferably 19 mass% or more, more preferably 50 mass% or more and further preferably 55 mass% or more, and preferably 80 mass% or less, more preferably 70 mass% or less and further preferably 68 mass% or less.

<52> The method for producing a sulfonate composition according to any of <1> to <51>, wherein the obtained sulfonate composition contains component (B) in an amount of preferably 20 mass% or more, more preferably 25 mass% or more and further preferably 45 mass% or more, and preferably 80 mass% or less and more preferably 45 mass% or less.

<53> The method for producing a sulfonate composition according to any of <1> to <52> including, reacting (A) a compound represented by the following general formula (1) in the presence of one or more solvents selected from (B) (B1) an organic solvent and (B2) water with (C) a sulfonating agent to obtain a mixture containing (S) a sulfonate represented by the following general formula (2), and removing an insoluble component from the mixture,

$$ROOC-(CH_2)_n-CH=CH-(CH_2)_m-COOR' \qquad (1)$$

wherein R and R' independently represent a group selected from alkyl groups with 10 carbons and alkenyl groups with 10 carbons; and n and m each represent a number of 0 or more and 18 or less, and a total of n and m is 0 or more and 18 or less,

$$ROOC-(CH_2)_p-CH(SO_3M)-CH_2-(CH_2)_q-COOR' \qquad (2)$$

wherein M represents a cation, and R and R' independently represent a group selected from alkyl groups with 10 carbons and alkenyl groups with 10 carbons; and p and q each represent a number of 0 or more and 18 or less, and a total of p and q is 0 or more and 18 or less,

wherein the mixture from which the removal of the insoluble component is made contains components (S), (B1) and (B2), wherein the mass ratio of the content of component (B1) to the total content of components (S), (B1) and (B2), (B1)/(S + B1 + B2), is 0.05 or more, and 0.65 or less and preferably 0.59 or less, the mass ratio of the content of component (B2) to the total content of components (S), (B1) and (B2), (B2)/(S + B1 + B2), is 0.03 or more, and 0.26 or less and preferably 0.20 or less, and preferably, a mass ratio of the content of component (B1) to the content of component (B2), (B1)/(B2), is 0.80 or more.

<54> The method for producing a sulfonate composition according to any of <1> to <53> including, reacting (A) a compound represented by the general formula (1) in the presence of one or more solvents selected from (B) (B1) an organic solvent and (B2) water with (C) a sulfonating agent to obtain a mixture containing (S) a sulfonate represented by the following general formula (2), depositing an insoluble component from the mixture, and removing the deposited component from the mixture,

$$ROOC-(CH_2)_n-CH=CH-(CH_2)_m-COOR' \qquad (1)$$

wherein R and R' independently represent a group selected from alkyl groups with 10 carbons and alkenyl groups with 10 carbons; and n and m each represent a number of 0 or more and 18 or less, and a total of n and m is 0 or more and 18 or less,

$$ROOC-(CH_2)_p-CH(SO_3M)-CH_2-(CH_2)_q-COOR' \qquad (2)$$

wherein M represents a cation, and R and R' independently represent a group selected from alkyl groups with 10 carbons and alkenyl groups with 10 carbons; and p and q each represent a number of 0 or more and 18 or less, and a total of p and q is 0 or more and 18 or less,

wherein the mixture from which the removal of the insoluble component is made contains components (S), (B1) and (B2), wherein the mass ratio of the content of component (B1) to the total content of components (S), (B1) and (B2), (B1)/(S + B1 + B2), is 0.05 or more, and 0.65 or less and preferably 0.59 or less, the mass ratio of the content of component (B2) to the total content of components (S), (B1) and (B2), (B2)/(S + B1 + B2), is 0.03 or more, and 0.26 or less and preferably 0.20 or less, and preferably, a mass ratio of the content of component (B1) to the content of component (B2), (B1)/(B2), is 0.80 or more.

<55> The method for producing a sulfonate composition according to any of <1> to <54>, wherein the insoluble component includes (D) an inorganic metal compound selected from unreacted component (C) and a hydrolyzed product of component (C).

<56> The method for producing a sulfonate composition according to any of <1> to <55>, wherein azeotropic distillation is not carried out in the removal of the insoluble component.

<57> The method for producing a sulfonate composition according to any of <1> to <56>, wherein organic solvent (B1) is not added to the sulfonate composition obtained by removing the insoluble component.

<58> The method for producing a sulfonate composition according to any of <1> to <57>, wherein a viscosity at 20°C of the mixture from which the removal of the insoluble component is made is preferably 600 mPa·s or less, more preferably 500 mPa·s or less, further preferably 470 mPa·s or less and furthermore preferably 450 mPa·s or less.

<59> The method for producing a sulfonate composition according to any of <1> to <58>, wherein a viscosity of the mixture from which the removal of the insoluble component is made is, for example, 0.1 mPa·s or more, 1 mPa·s or more and 10 mPa·s or more.

<60> The method for producing a sulfonate composition according to any of <1> to <59>, wherein a particle size of component (D) captured during an operation for removing the insoluble component is 10 μm or less and 5 μm or less.

<61> The method for producing a sulfonate composition according to any of <1> to <60>, wherein a particle size of component (D) captured during an operation for removing the insoluble component is preferably 0.1 μm or more and more preferably 1 μm or more.

<62> The method for producing a sulfonate composition according to any of <1> to <61>, wherein the content of component (D) in the sulfonate composition after the removal of the insoluble component is less than 1.6 parts by mass, more preferably 1.5 parts by mass or less and further preferably 1.0 parts by mass or less relative to 100 parts by mass of the content of component (S).

Examples

<Production example>

A maleic acid diester (di-(2-propylheptyl) maleic acid ester (DDM)) was obtained in conformance with Example 2 of US-A 2007/0214999. Further, a fumaric acid diester (di-(2-propylheptyl) fumaric acid ester (DDF)) was obtained by the same operations except that maleic anhydrite was replaced with fumaric acid.

<Step 1>

Next, the maleic acid diester (or fumaric acid diester), sodium disulfite, ion exchange water and propylene glycol (PG) were fed into a 1-L glass reaction vessel to achieve the compositions in Tables 1 to 3, and reacted in a publicly-known manner under the conditions shown in Tables 1 to 3. In each of examples 3 to 11 and 13 to 20 and comparative examples 1 to 2, the same dialkyl sulfonate (DASS) as one produced in that example or comparative example was prepared in advance and used as a reaction promoting agent. Note that, in the following explanation, DASS may include not only dialkyl sulfonates used as a reaction promoting agent but also those produced in Examples.

[Used reagent]

· Sodium disulfite: manufactured by FUJIFILM Wako Pure Chemical Corporation, a special grade reagent
· Propylene glycol: manufactured by FUJIFILM Wako Pure Chemical Corporation, a special grade reagent

<Step 2>

Next, the mixture containing a sulfonate obtained in step 1 was cooled to 50°C or less, and as necessary, propylene glycol and/or ion exchange water in the amounts shown in Tables 1 to 3 were added to the mixture.

<Step 3>

Next, while keeping the mixture at 55°C or less, unreacted component (C) and/or the hydrolyzed product of component (C) were oxidized with 30% hydrogen peroxide water. Further, a 35% aqueous sodium hydroxide solution was added to the mixture to adjust the pH to 5. When neutralized first, unreacted component (C) and/or the hydrolyzed product of component (C) were treated with 20% sodium carbonate and thereafter oxidized with 30% hydrogen peroxide water.

<Step 4>

Next, after Radiolite #900 (manufactured by Showa Chemical Industry Co., LTD.) was added to the mixture as a filter aid, the mixture was filtered under pressure at 0.4 MPa of nitrogen while the temperature was kept with warm water at 50°C circulated through a pressure filter with a jacket (inner capacity 2.9 liters, filtration area 0.0095 m$^2$). The captured particle size was 1 μm (a value listed in the filter aid's catalog).

(1) Measurement method

The content of each component in the sulfonate compositions obtained in examples 1 to 20 and comparative examples 1 and 2 was determined in the following manner. The content of each component was measured immediately after the filtration in step 4. Note that the content (mass%) of each component before the filtration in step 4, i.e., the content of each of DASS (S), PG (B1), sodium sulfate and water (B2) was calculated on the basis of a feed amount in step 1 or the like. Specifically, the content of DASS (S) was calculated in such a manner that an added amount of DASS which was added as necessary in step 1 was added to the amount of component (S) calculated on the basis of a feed amount of component (A) and a conversion rate in the tables. The content of PG was calculated on the basis of a feed amount in step 1 and an added amount in step 2. The content of sodium sulfate is the content of residual sodium disulfite expressed in terms of that of sodium sulfate, wherein the content of residual sodium disulfite was calculated on the basis of a feed amount of sodium disulfite in step 1 and a conversion rate in the tables. The content of water (B2) was calculated as the balance of the composition excluding DASS (S), PG (B1) and sodium sulfate. The results are shown in Tables 1 to 3.

(1-1) Mass percentage of sulfonate (DASS)

It was determined in accordance with the test method for synthetic detergents in JIS K 3362.

(1-2) Mass percentage of di-(2-propylheptyl) fumaric acid ester

Di-(2-propylheptyl) fumaric acid ester was quantified by gas chromatography.

(1-3) Mass percentage of propylene glycol

Propylene glycol was quantified by gas chromatography.

(1-4) Mass percentage of water

It was determined in accordance with Test methods for water content of chemical products in JIS K 0068.

(1-5) Mass percentage of sodium sulfate

Sodium sulfate was quantified using "automatic potentiometric titrator" manufactured by Kyoto Electronics Manufacturing Co., Ltd.

Further, in step 4, the viscosity at 20°C of the mixture to be filtered was measured with a B-type rotational viscometer (Brookfield viscometer, manufactured by Toki Sangyo Co., Ltd.) using No. 2 rotor.

(2) Evaluation method

(2-1) Evaluation of inorganic metal compound content of sulfonate composition

The amount of sodium sulfate in each prepared sulfonate composition was determined in the above manner. The results are shown in Tables 1 to 3. If each sulfonate composition immediately after step 4 contains sodium sulfate in an amount of 1.2 mass% or less and further less than 0.8 mass%, it can be considered as a sulfonate composition with a more reduced inorganic salt metal compound content.

(2-2) Rate of reduction of inorganic salts

The rate of reduction of inorganic salts was calculated by the formula below on the basis of the sodium sulfate contents (mass%) before and after the filtration in step 4. The results are shown in Tables 1 to 3. Note that, in the following formula, "before filtration" means the sodium sulfate content (mass%) before the filtration in step 4 and "after filtration" means the sodium sulfate content (mass%) after the filtration in step 4.

$$\text{Rate of reduction of inorganic salts (\%)} =$$

$$[(\text{before filtration} - \text{after filtration})/\text{before}$$

$$\text{filtration}] \times 100$$

(2-3) Proportion of inorganic salts relative to component (S)

Each sulfonate composition was evaluated on the basis of the parts by mass of sodium sulfate included in the sulfonate composition after step 4 relative to 100 parts by mass of component (S) included in the sulfonate composition.
The smaller the parts by mass of sodium sulfate relative to 100 parts by mass of component (S) is, the higher the degree of purity of the sulfonate composition is.

(2-4) Appearance evaluation

After 30 g of each sulfonate composition after step 4 was stored in a tightly-sealable glass container (50 mL) at 20°C for 24 hours, the appearance thereof was visually observed.

[Table 1]

| | | | | Example | | | | | |
|---|---|---|---|---|---|---|---|---|---|
| | | | | 1 | 2 | 3 | 4 | 5 | 6 |
| Step 1 | Component (A) | DDM (parts by mass) | | 100.0 | — | 100.0 | 100.0 | 100.0 | 100.0 |
| | | DDF (parts by mass) | | — | 100.0 | — | — | — | — |
| | Component (C) | Sodium disulfite (parts by mass) | | 26.4 | 26.4 | 26.4 | 26.4 | 27.6 | 28.8 |
| | Component (C)/component (A) (molar ratio) | | | 1.10 | 1.10 | 1.10 | 1.10 | 1.15 | 1.20 |
| | Component (B) | (B1) PG (parts by mass) | | 27.2 | 27.2 | 29.4 | 29.4 | 29.4 | 29.4 |
| | | (B2) Water (parts by mass) | | 4.8 | 4.8 | 7.8 | 7.7 | 7.7 | 7.7 |
| | | (B1)/(B2) (mass ratio) | | 5.7 | 5.7 | 3.8 | 3.8 | 3.8 | 3.8 |
| | Additive | DASS (parts by mass) | | — | — | 9.6 | 9.6 | 9.6 | 9.6 |
| | Reaction conditions | Reaction temperature [°C] | | 115°C | 115°C | 115°C | 115°C | 115°C | 115°C |
| | | Reaction pressure | | Atmospheric pressure | Atmospheric pressure | Atmospheric pressure | Atmospheric pressure | Atmospheric pressure | Atmospheric pressure |
| | | Reaction time [hr] | | 8 | 8 | 8 | 8 | 8 | 8 |
| | | Conversion rate [%] | | 92.3 | 94.4 | 96.2 | 96.2 | 96.2 | 95.5 |
| Step 2 | Component (B)[*1] | Added amount of PG (B1) (parts by mass) | | — | — | 1.8 | — | — | — |
| | | Content of PG (B1) (parts by mass) | | 27.2 | 27.2 | 31.2 | 29.4 | 29.4 | 29.4 |
| | | Added amount of water (B2) (parts by mass) | | 16.7 | 16.7 | 16.8 | 16.8 | 16.8 | 16.8 |
| | | Content of water (B2) (parts by mass) | | 21.5 | 21.5 | 24.6 | 24.5 | 24.5 | 24.5 |
| | | (B1)/(B2) (mass ratio) | | 1.27 | 1.27 | 1.27 | 1.20 | 1.20 | 1.20 |
| | | Temperature [°C] | | 25-30 | 25-30 | 25-30 | 35-40 | 25-30 | 35-40 |
| Step 3 | Order of operations | 1: Oxidation → neutralization 2: Neutralization → oxidation | | 1 | 1 | 1 | 1 | 1 | 1 |
| | Neutralization | Neutralizing agent | | NaOH | NaOH | NaOH | NaOH | NaOH | NaOH |
| | | Temperature [°C] | | 40-45 | 25-35 | 25-35 | 35-50 | 25-45 | 35-55 |
| | Oxidation | Oxidizing agent | | $H_2O_2$ | $H_2O_2$ | $H_2O_2$ | $H_2O_2$ | $H_2O_2$ | $H_2O_2$ |
| | | Temperature [°C] | | 35-45 | 30-40 | 35-40 | 35-50 | 40-45 | 50-55 |
| Step 4 | Method for removing insoluble component | | | Filtration | Filtration | Filtration | Filtration | Filtration | Filtration |
| | Temperature [°C] | | | 50 | 50 | 50 | 50 | 50 | 50 |
| | Mixture to be filtered | (S) DASS (mass%) | | 62.8 | 62.8 | 65.3 | 65.9 | 64.6 | 63.4 |
| | | (B1) PG (mass%) | | 14.6 | 14.6 | 15.6 | 14.8 | 14.5 | 14.2 |
| | | (B2) Water (mass%) | | 15.0 | 15.0 | 14.5 | 14.7 | 15.5 | 16.3 |
| | | Sodium sulfate (mass%) | | 3.4 | 3.4 | 2.5 | 2.5 | 3.3 | 4.1 |
| | | (B1)/(S + B1 + B2) (mass ratio) | | 0.158 | 0.158 | 0.164 | 0.155 | 0.153 | 0.151 |
| | | (B2)/(S + B1 + B2) (mass ratio) | | 0.162 | 0.162 | 0.152 | 0.154 | 0.164 | 0.174 |
| | | (B1)/(B2) (mass ratio) | | 0.973 | 0.973 | 1.08 | 1.01 | 0.935 | 0.871 |
| | | Content of PG (B1) (parts by mass)[*2] | | 27.2 | 27.2 | 31.2 | 29.4 | 29.4 | 29.4 |
| | | Content of water (B2) (parts by mass)[*2] | | 27.9 | 27.9 | 29.1 | 29.1 | 31.4 | 33.8 |
| | | Viscosity (mPa·s) at 20°C | | 413 | 413 | 413 | 413 | 413 | 413 |
| | Composition after step 4 | (S) DASS (mass%) | | 69.6 | 69.2 | 69.7 | 69.7 | 69.1 | 68.7 |
| | | (B1) PG (mass%) | | 16.4 | 16.2 | 16.5 | 16.5 | 16.3 | 16.3 |
| | | (B2) Water (mass%) | | 11.2 | 11.2 | 12.3 | 12.3 | 12.9 | 12.3 |
| | | Sodium sulfate (mass%) | | 0.2 | 0.3 | 0.4 | 0.4 | 0.3 | 0.2 |
| | Evaluations | Rate of reduction of inorganic salts (%) | | 94 | 92 | 85 | 85 | 90 | 95 |
| | | Appearance at 20°C | | Uniform and transparent | Uniform and transparent | Uniform and transparent | Uniform and transparent | Uniform and transparent | Uniform and transparent |
| | | Content of (D) (parts by mass relative to 100 parts by mass of (S)) | | 0.3 | 0.4 | 0.5 | 0.5 | 0.5 | 0.3 |

The parts by mass (added amounts or contents) of components (B1) and (B2) marked with *1 and *2 in Table 1 each represent the parts by mass of component (B1) or (B2) relative to 100 parts by mass of component (A), a raw material, before the sulfonation reaction. The same applies to *1 and *2 in the following Tables 2 and 3.

[Table 2]

| | | | Example | | | | | |
|---|---|---|---|---|---|---|---|---|
| | | | 7 | 8 | 9 | 10 | 11 | 12 |
| Step 1 | Component (A) | DDM (parts by mass) | 100.0 | 100.0 | 100.0 | 100.0 | 100.0 | 100.0 |
| | | DDF (parts by mass) | — | — | — | — | — | — |
| | Component (C) | Sodium disulfite (parts by mass) | 26.7 | 26.4 | 26.4 | 26.4 | 26.4 | 26.4 |
| | Component (C)/component (A) (molar ratio) | | 1.10 | 1.10 | 1.10 | 1.10 | 1.10 | 1.10 |
| | Component (B) | (B1) PG (parts by mass) | 29.4 | 59.3 | 45.6 | 29.4 | 18.2 | 27.4 |
| | | (B2) Water (parts by mass) | 7.8 | 24.7 | 21.9 | 7.7 | 14.0 | 4.8 |
| | | (B1)/(B2) (mass ratio) | 3.8 | 2.4 | 2.1 | 3.8 | 1.3 | 5.7 |
| | Additive | DASS (parts by mass) | 9.6 | 9.6 | 9.6 | 9.6 | 9.6 | — |
| | Reaction conditions | Reaction temperature [°C] | 105°C | 115°C | 115°C | 115°C | 115°C | 115°C |
| | | Reaction pressure | Atmospheric pressure | Atmospheric pressure | Atmospheric pressure | Atmospheric pressure | Atmospheric pressure | Atmospheric pressure |
| | | Reaction time [hr] | 8 | 8 | 8 | 8 | 8 | 10 |
| | | Conversion rate [%] | 93.9 | 97.4 | 97.4 | 97.4 | 95.0 | 92.6 |
| Step 2 | Component (B)[*1] | Added amount of PG (B1) (parts by mass) | 1.9 | — | — | — | — | 1.9 |
| | | Content of PG (B1) (parts by mass) | 31.3 | 59.3 | 45.6 | 29.4 | 18.2 | 29.3 |
| | | Added amount of water (B2) (parts by mass) | 17.2 | — | — | 16.7 | 23.2 | 25.6 |
| | | Content of water (B2) (parts by mass) | 25.0 | 24.7 | 21.9 | 24.4 | 37.2 | 30.4 |
| | | (B1)/(B2) (mass ratio) | 1.25 | 2.41 | 2.08 | 1.20 | 0.49 | 0.96 |
| | | Temperature [°C] | 25-30 | — | — | 30-35 | 25-30 | 25-30 |
| Step 3 | Order of operations | 1: Oxidation → neutralization<br>2: Neutralization → oxidation | 1 | 1 | 1 | 2 | 1 | 1 |
| | Neutralization | Neutralizing agent | NaOH | NaOH | NaOH | Na₂CO₃ | NaOH | NaOH |
| | | Temperature [°C] | 25-50 | 20-30 | 25-30 | 30-35 | 25-30 | 25-50 |
| | Oxidation | Oxidizing agent | H₂O₂ | H₂O₂ | H₂O₂ | H₂O₂ | H₂O₂ | H₂O₂ |
| | | Temperature [°C] | 35-45 | 25-30 | 25-35 | 30-45 | 25-30 | 45-50 |
| Step 4 | Method for removing insoluble component | | Filtration | Filtration | Filtration | Filtration | Filtration | Filtration |
| | Temperature [°C] | | 50 | 50 | 50 | 50 | 50 | 50 |
| | Mixture to be filtered | (S) DASS (mass%) | 62.8 | 58.1 | 61.4 | 66.2 | 63.9 | 59.0 |
| | | (B1) PG (mass%) | 15.4 | 26.1 | 21.5 | 14.8 | 9.00 | 14.8 |
| | | (B2) Water (mass%) | 15.4 | 12.6 | 12.6 | 14.5 | 21.4 | 18.8 |
| | | Sodium sulfate (mass%) | 3.1 | 2.0 | 2.4 | 2.5 | 3.0 | 3.3 |
| | | (B1)/(S + B1 + B2) (mass ratio) | 0.165 | 0.270 | 0.225 | 0.155 | 0.095 | 0.160 |
| | | (B2)/(S + B1 + B2) (mass ratio) | 0.165 | 0.130 | 0.132 | 0.152 | 0.227 | 0.203 |
| | | (B1)/(B2) (mass ratio) | 1.00 | 2.07 | 1.71 | 1.02 | 0.421 | 0.787 |
| | | Content of PG (B1) (parts by mass)[*2] | 31.3 | 59.3 | 45.6 | 29.4 | 18.2 | 29.2 |
| | | Content of water (B2) (parts by mass)[*2] | 31.3 | 28.6 | 26.6 | 28.8 | 43.2 | 37.1 |
| | | Viscosity (mPa·s) at 20°C | 275 | 145 | 176 | 336 | 466 | 279 |
| | Composition after step 4 | (S) DASS (mass%) | 66.3 | 60.4 | 67.1 | 69.9 | 71.0 | 63.8 |
| | | (B1) PG (mass%) | 16.5 | 26.9 | 21.1 | 13.8 | 9.5 | 14.8 |
| | | (B2) Water (mass%) | 15.6 | 10.5 | 9.3 | 14.1 | 15.3 | 16.9 |
| | | Sodium sulfate (mass%) | 0.4 | 0.1 | 0.1 | 0.5 | 1.1 | 0.9 |
| | Evaluations | Rate of reduction of inorganic salts (%) | 88 | 96 | 96 | 80 | 63 | 73 |
| | | Appearance at 20°C | Uniform and transparent | Uniform and transparent | Uniform and transparent | Uniform and transparent | Uniform and transparent | Uniform and transparent |
| | | Content of (D) (parts by mass relative to 100 parts by mass of (S)) | 0.5 | 0.1 | 0.1 | 0.7 | 1.5 | 1.4 |

# EP 4 265 595 A1

[Table 3]

| | | | Example | | | | | | | | Comparative example | |
|---|---|---|---|---|---|---|---|---|---|---|---|---|
| | | | 13 | 14 | 15 | 16 | 17 | 18 | 19 | 20 | 1 | 2 |
| Step 1 | Component (A) | DDM (parts by mass) | 100.0 | 100.0 | 100.0 | 100.0 | 100.0 | 100.0 | 100.0 | 100.0 | 100.0 | 100.0 |
| | | DDF (parts by mass) | — | — | — | — | — | — | — | — | — | — |
| | Component (C) | Sodium disulfite (parts by mass) | 27.5 | 27.5 | 27.5 | 27.6 | 27.6 | 27.5 | 27.6 | 27.5 | 27.5 | 27.5 |
| | Component (C)/component (A) (molar ratio) | | 1.15 | 1.15 | 1.15 | 1.15 | 1.15 | 1.15 | 1.15 | 1.15 | 1.15 | 1.15 |
| | Component (B) | (B1) PG (parts by mass) | 29.4 | 29.4 | 29.4 | 29.9 | 29.9 | 29.4 | 29.9 | 29.4 | 29.4 | 29.4 |
| | | (B2) Water (parts by mass) | 5.8 | 5.8 | 5.8 | 5.5 | 5.5 | 5.8 | 5.5 | 5.8 | 5.8 | 5.8 |
| | | (B1)/(B2) (mass ratio) | 5.1 | 5.1 | 5.1 | 5.4 | 5.4 | 5.1 | 5.4 | 5.1 | 5.1 | 5.1 |
| | Additive | DASS (parts by mass) | 9.4 | 9.4 | 9.4 | 9.6 | 9.6 | 9.4 | 9.6 | 9.4 | 9.4 | 9.4 |
| | Reaction conditions | Reaction temperature [°C] | 105°C | 105°C | 105°C | 105°C | 105°C | 105°C | 105°C | 105°C | 105°C | 105°C |
| | | Reaction pressure | Atmospheric pressure | Atmospheric pressure | Atmospheric pressure | Atmospheric pressure | Atmospheric pressure | Atmospheric pressure | Atmospheric pressure | Atmospheric pressure | Atmospheric pressure | Atmospheric pressure |
| | | Reaction time [hr] | 8 | 8 | 8 | 8 | 8 | 8 | 8 | 8 | 8 | 8 |
| | | Conversion rate [%] | 97.3 | 97.3 | 97.3 | 97.3 | 97.3 | 97.3 | 97.3 | 97.3 | 97.3 | 97.3 |
| Step 2 | Component (B)*1 | Added amount of PG (B1) (parts by mass) | 213 | 315 | 75.4 | 105 | 50.3 | 262 | 343 | 168 | 86.1 | 36.2 |
| | | Content of PG (B1) (parts by mass) | 242 | 344 | 105 | 135 | 80.3 | 291 | 373 | 198 | 115 | 65.6 |
| | | Added amount of water (B2) (parts by mass) | 44.8 | 32.1 | 5.5 | 37.5 | 23.4 | 85.0 | 140 | 88.3 | 61.9 | 44.2 |
| | | Content of water (B2) (parts by mass) | 50.6 | 37.9 | 11.3 | 43.0 | 28.9 | 90.8 | 146 | 94.1 | 67.7 | 50.1 |
| | | (B1)/(B2) (mass ratio) | 4.78 | 9.07 | 9.30 | 3.13 | 2.78 | 3.21 | 2.56 | 2.10 | 1.70 | 1.31 |
| | | Temperature [°C] | 35-40 | 35-40 | 35-40 | 35-40 | 35-40 | 35-40 | 35-40 | 35-40 | 35-40 | 35-40 |
| Step 3 | Order of operations | 1: Oxidation → neutralization 2: Neutralization → oxidation | 1 | 1 | 1 | 1 | 1 | 1 | 1 | 1 | 1 | 1 |
| | Neutralization | Neutralizing agent | NaOH | NaOH | NaOH | NaOH | NaOH | NaOH | NaOH | NaOH | NaOH | NaOH |
| | | Temperature [°C] | 35-55 | 35-55 | 35-55 | 35-55 | 35-55 | 35-55 | 35-55 | 35-55 | 35-55 | 35-55 |
| | Oxidation | Oxidizing agent | $H_2O_2$ | $H_2O_2$ | $H_2O_2$ | $H_2O_2$ | $H_2O_2$ | $H_2O_2$ | $H_2O_2$ | $H_2O_2$ | $H_2O_2$ | $H_2O_2$ |
| | | Temperature [°C] | 50-55 | 50-55 | 50-55 | 50-55 | 50-55 | 50-55 | 50-55 | 50-55 | 50-55 | 50-55 |
| Step 4 | Method for removing insoluble component | | Filtration | Filtration | Filtration | Filtration | Filtration | Filtration | Filtration | Filtration | Filtration | Filtration |
| | Temperature [°C] | | 50 | 50 | 50 | 50 | 50 | 50 | 50 | 50 | 50 | 50 |
| | Mixture to be filtered | (S) DASS (mass%) | 29.0 | 24.2 | 47.3 | 38.2 | 47.6 | 24.3 | 19.2 | 29.1 | 38.2 | 47.4 |
| | | (B1) PG (mass%) | 53.1 | 63.1 | 37.5 | 39.6 | 29.8 | 53.4 | 54.6 | 43.4 | 33.3 | 23.5 |
| | | (B2) Water (mass%) | 15.9 | 11.0 | 11.8 | 19.6 | 19.4 | 20.7 | 24.8 | 25.5 | 25.8 | 25.8 |
| | | Sodium sulfate (mass%) | 1.4 | 1.2 | 2.3 | 1.9 | 2.3 | 1.2 | 0.9 | 1.4 | 1.9 | 2.3 |
| | | (B1)/(S + B1 + B2) (mass ratio) | 0.542 | 0.642 | 0.388 | 0.406 | 0.308 | 0.543 | 0.554 | 0.443 | 0.342 | 0.243 |
| | | (B2)/(S + B1 + B2) (mass ratio) | 0.162 | 0.111 | 0.122 | 0.201 | 0.200 | 0.210 | 0.251 | 0.260 | 0.265 | 0.266 |
| | | (B1)/(B2) (mass ratio) | 3.34 | 5.76 | 3.17 | 2.02 | 1.54 | 2.58 | 2.20 | 1.71 | 1.29 | 0.91 |
| | | Content of PG (B1) (parts by mass)*2 | 242 | 344 | 105 | 137 | 82.8 | 291 | 376 | 198 | 115 | 65.6 |
| | | Content of water (B2) (parts by mass)*2 | 72.4 | 59.7 | 33.1 | 68.0 | 53.9 | 113 | 171 | 116 | 89.5 | 71.9 |
| | | Viscosity (mPa·s) at 20°C | 57 | 56 | 131 | 69 | 123 | 45 | 29 | 53 | 49 | 127 |
| | Composition after step 4 | (S) DASS (mass%) | 29.9 | 24.8 | 49.6 | 38.6 | 49.1 | 25.0 | 19.3 | 29.7 | 39.3 | 49.7 |
| | | (B1) PG (mass%) | 54.1 | 64.1 | 38.1 | 40.2 | 30.4 | 54.2 | 54.3 | 43.8 | 33.8 | 22.9 |
| | | (B2) Water (mass%) | 15.6 | 10.6 | 11.1 | 19.6 | 18.4 | 19.9 | 25.0 | 25.0 | 26.4 | 25.6 |
| | | Sodium sulfate (mass%) | 0.06 | 0.03 | 0.07 | 0.2 | 0.2 | 0.2 | 0.2 | 0.5 | 0.8 | 1.2 |
| | Evaluations | Rate of reduction of inorganic salts (%) | 96 | 97 | 97 | 92 | 90 | 87 | 80 | 67 | 58 | 47 |
| | | Appearance at 20°C | Uniform and transparent | Uniform and transparent | Uniform and transparent | Uniform and transparent | Uniform and transparent | Uniform and transparent | Uniform and transparent | Uniform and transparent | Separated and whitish | Separated and whitish |
| | | Content of (D) (parts by mass relative to 100 parts by mass of (S)) | 0.20 | 0.12 | 0.14 | 0.39 | 0.47 | 0.60 | 0.99 | 1.58 | 2.01 | 2.47 |

Tables 1 to 3 show that the method for producing a sulfonate composition of the present invention can efficiently remove insoluble components (for example, inorganic metal compounds such as sodium sulfate or the like) from the sulfonate composition.

Further, the method for producing a sulfonate composition of the present invention can produce a sulfonate composition including a high concentration of sulfonate (S) represented by the general formula (2) and effectively reduce the content of insoluble components (for example, inorganic metal compounds such as sodium sulfate or the like) relative to the content of component (S) in the sulfonate composition.

**Claims**

1.  A method for producing a sulfonate composition comprising, reacting (A) a compound represented by the following general formula (1) [hereinafter referred to as component (A)] in the presence of one or more solvents selected from (B) (B1) an organic solvent [hereinafter referred to as component (B1)] and (B2) water [hereinafter referred to as component (B2)] with (C) a sulfonating agent [hereinafter referred to as component (C)] to obtain a mixture containing (S) a sulfonate represented by the following general formula (2) [hereinafter referred to as component (S)], and removing an insoluble component from the mixture,

    $$ROOC\text{-}(CH_2)_n\text{-}CH{=}CH\text{-}(CH_2)_m\text{-}COOR' \qquad (1)$$

    wherein R and R' independently represent a group selected from alkyl groups with 10 carbons and alkenyl groups with 10 carbons; and n and m each represent a number of 0 or more and 18 or less, and a total of n and m is 0 or more and 18 or less,

    $$ROOC\text{-}(CH_2)_p\text{-}CH(SO_3M)\text{-}CH_2\text{-}(CH_2)_q\text{-}COOR' \qquad (2)$$

    wherein M represents a cation, and R and R' independently represent a group selected from alkyl groups with 10 carbons and alkenyl groups with 10 carbons; and p and q each represent a number of 0 or more and 18 or less, and a total of p and q is 0 or more and 18 or less,
    wherein the mixture from which the removal of the insoluble component is made comprises the components (S), (B1) and (B2), wherein a mass ratio of the content of the component (B1) to a total content of the components (S), (B1) and (B2), (B1)/(S + B1 + B2), is 0.05 or more and 0.65 or less, and a mass ratio of the content of the component (B2) to the total content of the components (S), (B1) and (B2), (B2)/(S + B1 + B2), is 0.03 or more and 0.26 or less.

2.  The method for producing a sulfonate composition according to claim 1, wherein the component (A) is a compound of the general formula (1) in which R and R' independently represent a group selected from 2-propylheptyl group, 4-methyl 2-propylhexyl group and 5-methyl 2-propylhexyl group.

3.  The method for producing a sulfonate composition according to claim 1 or 2, wherein one or more solvents selected from the components (B1) and (B2) are added to the mixture, and the insoluble component is thereafter removed from the mixture.

4.  The method for producing a sulfonate composition according to any one of claims 1 to 3, wherein in the sulfonate composition, the content of the component (B1) is 5 mass% or more and 65 mass% or less, and the content of the component (B2) is 3 mass% or more and 25 mass% or less.

5.  The method for producing a sulfonate composition according to any one of claims 1 to 4, wherein the component (B1) is at least one selected from a monohydric alcohol and a polyhydric alcohol.

6.  The method for producing a sulfonate composition according to any one of claims 1 to 5, wherein the component (C) is at least one compound selected from $MHSO_3$, $M_2SO_3$ and $M_2S_2O_5$, wherein M represents a cation.

7.  The method for producing a sulfonate composition according to any one of claims 1 to 6, wherein the insoluble component comprises (D) an inorganic metal compound selected from an unreacted component (C) and a hydrolyzed product of the component (C).

8.  The method for producing a sulfonate composition according to any one of claims 1 to 7, wherein the component (C) is used at a ratio of 1.00 mol or more and 1.20 mol or less relative to 1 mol of the component (A).

9.  The method for producing a sulfonate composition according to any one of claims 1 to 8, wherein the component (A) is reacted with the component (C) at a reaction temperature of 100°C or more and less than 125°C and at a normal pressure or under pressure.

10. The method for producing a sulfonate composition according to any one of claims 1 to 9, wherein an unreacted component (C) in the mixture is neutralized and/or oxidized before removing the insoluble component.

11. The method for producing a sulfonate composition according to any one of claims 1 to 10, wherein a sulfonate obtained by reacting the component (A) with the component (C) is prepared separately, and the component (A) is reacted with the component (C) under the coexistence of the sulfonate.

12. The method for producing a sulfonate composition according to any one of claims 1 to 11, wherein the component (B1) is one or more compounds selected from methanol, ethanol, 1-propanol, 1-butanol, 1-pentanol, 1-hexanol, 1-octanol, 1-nonanol, 2-nonanol, 5-nonanol, 1-decanol, 1-dodecanol, 3,5,5-trimethylhexan-1-ol, 2,6-dimethyl-4-heptanol, 2-propylheptanol, 4-methyl 2-propylhexanol and 5-methyl 2-propylhexanol.

13. The method for producing a sulfonate composition according to any one of claims 1 to 11, wherein the component (B1) is one or more compounds selected from ethylene glycol, propylene glycol, 1,3-butanediol, 1,4-butanediol, 1,5-pentanediol, 1,6-hexanediol, 1,10-decanediol, diethylene glycol, triethylene glycol, tetraethylene glycol, dipropylene glycol, tripropylene glycol, glycerin, xylitol and sorbitol.

14. The method for producing a sulfonate composition according to any one of claims 1 to 11, wherein the component (B1) is one or more compounds selected from ethanol, 1-butanol, 1-propanol, glycerin and propylene glycol.

15. The method for producing a sulfonate composition according to any one of claims 1 to 14, wherein the content of the component (D) in the sulfonate composition after the removal of the insoluble component is less than 1.6 parts by mass relative to 100 parts by mass of the content of the component (S).

## INTERNATIONAL SEARCH REPORT

| International application No. |
|---|
| **PCT/JP2021/047178** |

**A. CLASSIFICATION OF SUBJECT MATTER**

*C07C 303/44*(2006.01)i; *C07C 303/20*(2006.01)i; *C07C 303/32*(2006.01)i; *C07C 309/17*(2006.01)i; *C09K 23/10*(2022.01)i; *C11D 1/28*(2006.01)i; *C11D 3/20*(2006.01)i; *C11D 3/43*(2006.01)i

FI: C07C303/44; B01F17/10; C07C303/20; C07C303/32; C07C309/17; C11D1/28; C11D3/20; C11D3/43

According to International Patent Classification (IPC) or to both national classification and IPC

**B. FIELDS SEARCHED**

Minimum documentation searched (classification system followed by classification symbols)

C07C303/00-303/46; C07C309/00-309/89; C09K23/10; C11D1/00-19/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | JP 2018-529989 A (BASF SE) 11 October 2018 (2018-10-11) paragraph [0261] | 1-15 |
| A | JP 8-337567 A (NIPPON OIL & FATS CO LTD) 24 December 1996 (1996-12-24) entire text | 1-15 |
| A | US 2007/0214999 A1 (MEYER, Joachim) 20 September 2007 (2007-09-20) entire text | 1-15 |
| A | JP 60-112752 A (UNILEVER NV) 19 June 1985 (1985-06-19) entire text | 1-15 |

☐ Further documents are listed in the continuation of Box C.      ☑ See patent family annex.

\* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **17 February 2022** | **01 March 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

INTERNATIONAL SEARCH REPORT
Information on patent family members

International application No.

**PCT/JP2021/047178**

| Patent document cited in search report | | | Publication date (day/month/year) | Patent family member(s) | | | Publication date (day/month/year) |
|---|---|---|---|---|---|---|---|
| JP | 2018-529989 | A | 11 October 2018 | US 2018/0201885 A1 paragraph [0670] WO 2017/009068 A1 CN 107849493 A KR 10-2018-0033229 A | | | |
| JP | 8-337567 | A | 24 December 1996 | (Family: none) | | | |
| US | 2007/0214999 | A1 | 20 September 2007 | EP 1829859 A2 entire text DE 102006009971 A | | | |
| JP | 60-112752 | A | 19 June 1985 | GB 8329074 A entire text | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070214999 A
- JP 2002224552 A
- JP H9500884 A
- JP H8337567 A
- JP 2018162446 A